# EUROPÄISCHE PATENTSCHRIFT

(11) **EP 3 057 580 B1**
(45) Veröffentlichungstag und Bekanntmachung des Hinweises auf die Patenterteilung: **24.02.2021**
(21) Anmeldenummer: 14783642.3
(22) Anmeldetag: 13.10.2014
(51) Int. Cl.: A61K 9/20, A61K 9/28, A61K 31/5377, A61P 9/04, A61P 13/12, A61P 7/06

(54) **PHARMAZEUTISCHE DARREICHUNGSFORMEN ENTHALTEND NATRIUM-1-[6-(MORPHOLIN-4-YL)PYRIMIDIN-4-YL]-4-(1H-1,2,3-TRIAZOL-1-YL)-1H-PYRAZOL-5-OLAT**
PHARMACEUTICAL DOSAGE FORMS CONTAINING SODIUM-1-[6-(MORPHOLIN-4-YL)PYRIMIDIN-4-YL]-4-(1H-1,2,3-TRIAZOL-1-YL)-1H-PYRAZOL-5-OLATE
FORMES GALÉNIQUES PHARMACEUTIQUES CONTENANT DU SODIUM 1-[6-{MORPHOLINE-4-YL)PYRIMIDINE-4-YL]-4-(1H-1,2,3- TRIAZOL-1-YL)-1H-PYRAZOL-5-OLATE

(30) Priorität: 17.10.2013 EP 13189145
(43) Veröffentlichungstag der Anmeldung: 24.08.2016
(73) Patentinhaber: Bayer Pharma Aktiengesellschaft, 13353 Berlin (DE)
(72) Erfinder: NEUMANN, Heike, 42117 Wuppertal (DE); BENKE, Klaus, 51465 Bergisch Gladbach (DE); FORMELL, Michael, 16548 Glienicke (DE); WINTER, Gabriele, 16548 Glienicke (DE)
(74) Vertreter: BIP Patents
(86) Internationale Anmeldenummer: PCT/EP2014/071855
(87) Internationale Veröffentlichungsnummer: WO 2015/055564

(56) Entgegenhaltungen:
- WO-A1-2012/065967

## Beschreibung

Die vorliegende Erfindung betrifft feste, oral applizierbare pharmazeutische Darreichungsformen enthaltend Natrium-1-[6-(morpholin-4-yl)pyrimidin-4-yl]-4-(1H-1,2,3-triazol-1-yl)-1H-pyrazol-5-olat (Wirkstoff (I)), dadurch gekennzeichnet, dass der Wirkstoff (I) freigegeben wird, sowie Verfahren zu ihrer Herstellung, ihrer Anwendung als Arzneimittel, sowie ihrer Verwendung zur Prophylaxe, Sekundärprophylaxe oder Behandlung von Erkrankungen, insbesondere von Herz-Kreislauf-Erkrankungen, Herzinsuffizienz, Anämie, chronischen Nierenerkrankungen und Niereninsuffizienz.

Die Verbindung der Formel (II), 1-[6-(Morpholin-4-yl)pyrimidin-4-yl]-4-(1H-1,2,3-triazol-1-yl)-1H-pyrazol-5-ol (Enol-Form; Formel (IIa)) bzw. 2-[6-(Morpholin-4-yl)pyrimidin-4-yl]-4-(1H-1,2,3-triazol-1-yl)-1,2-dihydro-3H-pyrazol-3-on (Keto-Form; Formel (IIb)), ist aus WO 2008/067871 bekannt.

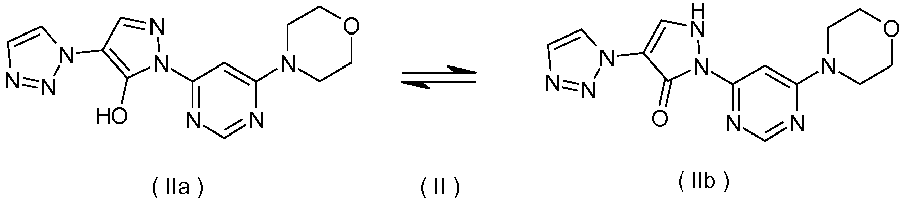

Die Verbindung der Formel (II) wirkt als Hemmstoff der HIF-Prolyl-4-Hydroxylasen und führt aufgrund dieses spezifischen Wirkmechanismus *in vivo* nach parenteraler oder oraler Verabreichung die Induktion von HIF-Zielgenen, wie z.B. Erythropoetin, und der hierdurch verursachten biologischen Prozesse, wie z.B. Erythropoese, herbei.

Der Wirkstoff (I), Natrium-1-[6-(morpholin-4-yl)pyrimidin-4-yl]-4-(1H-1,2,3-triazol-1-yl)-1H-pyrazol-5-olat, 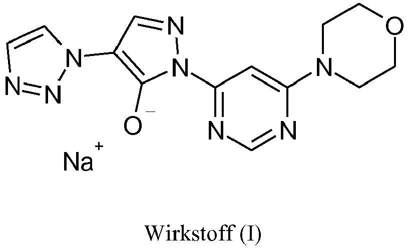 ist das Natriumsalz der Verbindung der Formel (II) und zeigt eine signifikant höhere Stabilität gegenüber der Aufnahme beziehungsweise Abgabe von Wasser bei schwankender Luftfeuchtigkeit. Das Natrium-1-[6-(morpholin-4-yl)pyrimidin-4-yl]-4-(1H-1,2,3-triazol-1-yl)-1H-pyrazol-5-olat (Wirkstoff (I)) ist aus WO 2012/065967 bekannt. Wenn im Folgenden von Wirkstoff (I) die Rede ist, so ist dabei die Kristallmodifikation I von Natrium-1-[6-(morpholin-4-yl)pyrimidin-4-yl]-4-(1H-1,2,3-triazol-1-yl)-1H-pyrazol-5-olat (I) gemeint. Weiterhin offenbart WO 2012/065967 eine feste, oral applizierbare pharmazeutische Darreichungsform enthaltend Natrium-1-[6-(morpholin-4-yl)pyrimidin-4-yl]-4-(1H-1,2,3-triazol-1-yl)-1H-pyrazol-5-olat (Wirkstoff (I)). Die Merkmale a)-d) der vorliegenden Erfindung sind in WO 2012/065967 nicht offenbart.

Für die Entwicklung einer festen, oral applizierbaren pharmazeutischen Darreichungsform wird daher Natrium-1-[6-(morpholin-4-yl)pyrimidin-4-yl]-4-(1H-1,2,3-triazol-1-yl)-1H-pyrazol-5-olat (Wirkstoff (I)) eingesetzt, welches diese Hygroskopizität nicht aufweist.

Bei Krankheiten, die über einen längeren Zeitraum behandelt werden müssen, oder zur längerfristigen Prophylaxe von Krankheiten ist es wünschenswert, die Häufigkeit der Einnahme von Medikamenten so gering wie möglich und die Tablettengröße so klein wie möglich zu halten. Dies ist nicht nur bequemer für den Patienten, sondern es erhöht auch die Behandlungssicherheit, indem es die Nachteile unregelmäßiger Einnahmen vermindert (Verbesserung der Compliance). Um die Compliance insbesondere bei älteren Patienten zu erhöhen, sollten die Tabletten insbesondere im Hinblick auf die höheren Dosisstärken möglichst klein sein, d.h. eine hohe Wirkstoffkonzentration aufweisen.

Im Laufe der Entwicklung wurde festgestellt, dass bei der Erhöhung der Wirkstoffkonzentration des Wirkstoffes (I) in Tabletten, wobei die Tabletten nach dem Fachmann bekannten Standardmethoden hergestellt wurden, sich die Freisetzungsrate des Wirkstoffes (I) aus den Tabletten verschlechterte.

Ziel der Entwicklung war es daher, feste, oral applizierbare pharmazeutische Darreichungsformen enthaltend Natrium-1-[6-(morpholin-4-yl)pyrimidin-4-yl]-4-(1H-1,2,3-triazol-1-yl)-1H-pyrazol-5-olat (Wirkstoff (I)) für hohe Wirkstoffkonzentration zu identifizieren, um auch bei hohen Wirkstoffkonzentrationen möglichst kleine z. B. Tabletten zu erhalten.

Überraschenderweise haben die Hilfsstoffe der pharmazeutischen Darreichungsformen einen erheblichen Einfluss auf die Freisetzung direkt nach Herstellung der Tabletten, trotz der guten Wasserlöslichkeit von Wirkstoff (I). Es kam mit üblichen Standardformulierungen im Festkörperzustand (solid state) zu Umsalzungen des Wirkstoffes (I) in schwerlösliche Salz-Formen mit der Konsequenz, dass sich die Freisetzungsergebnisse direkt nach der Herstellung von Tabletten und nach dem Stresstest von Tabletten deutlich verschlechterten.

Der Einsatz von Laktose als Hilfsstoff, beispielsweise im Tablettenkern, ist überraschenderweise ungeeignet.

Weiterhin zeigt der Wirkstoff (I) bei hohen Wirkstoffkonzentrationen eine physikalische Inkompatibilität mit im Filmüberzug verwendetem Polyethylenglykol.

Durch die vorliegende Erfindung ist die Bereitstellung einer stabilen pharmazeutischen Darreichungsform möglich, die den Wirkstoff (I) einerseits in für dessen pharmazeutische Wirkung ausreichender Menge enthält und andererseits den Wirkstoff (I) schnell freisetzt.

Bei der Formulierungsentwicklung sind die physikalisch-chemischen Charakteristika in Kombination mit den besonderen biologischen Eigenschaften des Wirkstoffes (I) zu berücksichtigen. Zu den physikalisch-chemischen Charakteristika zählt beispielsweise das Umsalzen des Wirkstoffes (I) [= Natrium-Salz] in schwerlösliche Salze.

Es wurde gefunden, dass Wirkstoff (I) in Gegenwart 2- und 3-wertiger Kationen dazu neigt, schwerlösliche Salze zu bilden, deren Löslichkeit nur noch 1/100 bzw. 1/10.000 der Löslichkeit des Natriumsalzes beträgt.

Löslichkeit verschiedener Wirkstoffsalze der Verbindung der Formel (II):

| **Salz** | **Löslichkeit in Wasser bei 25°C [mg/100mL]** |
|---|---|
| Natrium-Salz (Wirkstoff (I)) | 2625 |
| Magnesium-Salz | 4 |
| Eisen(II)-Salz | 0.2 |
| Calcium-Salz | 27 |
| Eisen(III)-Salz | 19 |
| Aluminium-Salz | 6 |

Das hat zur Konsequenz, dass bei Umsalzung des Wirkstoffes (I) im Festkörperzustand (solid state) sich die Freisetzungsergebnisse des Wirkstoffes (I) nach Lagerung von z. B. Tabletten deutlich verschlechterten. Diese Verschlechterung fällt besonders bei hohen Wirkstoffkonzentrationen auf.

Das Umsalzen des Wirkstoff (I) muss bei der Entwicklung von festen, oral applizierbaren pharmazeutischen Darreichungsformen enthaltend den Wirkstoff (I) verhindert werden.

Im Rahmen der vorliegenden Erfindung erfolgt die Konzentrationsbestimmung des Wirkstoffes (I) über den Wirkstoff (I) (Natrium-Salz).

Einer Tablettendosis von 100 mg, d.h. 100 mg der Verbindung der Formel (II), entsprechen 107 mg des Wirkstoffes (I) (Natrium-Salz).

Im Rahmen der vorliegenden Erfindung bedeutet eine hohe Wirkstoffkonzentration eine Konzentration des Wirkstoffes (I) ≥ 10% bezogen auf die Gesamtmasse der Formulierung.

Gegenstand der vorliegenden Erfindung sind feste, oral applizierbare pharmazeutische Darreichungsformen enthaltend Natrium-1-[6-(morpholin-4-yl)pyrimidin-4-yl]-4-(1H-1,2,3-triazol-1-yl)-1H-pyrazol-5-olat (Wirkstoff (I)), dadurch gekennzeichnet, dass
(a) keine Hilfsstoffe mit 2- und/oder 3-wertigen Kationen enthalten sind,
(b) keine Laktose enthalten ist,
(c) die Konzentration des Wirkstoffes (I) ≥ 10% bezogen auf die Gesamtmasse der Formulierung beträgt, und
(d) sofern die Darreichungsform einen Filmüberzug hat, dieser Filmüberzug kein Polyethylenglykol enthält.

Im Rahmen der vorliegenden Erfindung sind Hilfsstoffe Bindemittel, Füllstoffe und Trockenbindemittel, Zerfallsförderer und Schmiermittel.

Ein Hilfsstoff mit 2- und/oder 3-wertigen Kationen ist beispielsweise Magnesiumstearat.

Im Rahmen der vorliegenden Erfindung sind in den festen, oral applizierbaren pharmazeutischen Darreichungsformen als Hilfsstoffe mindestens ein Füllstoff und mindestens ein Schmiermittel enthalten.

Der Filmüberzug enthält Lackier- und/oder Filmbildemittel und/oder Farbzusätze/Pigmente. Diese Bestandteile des Filmüberzugs können gegebenenfalls 2- und/oder 3-wertigen Kationen enthalten.

Farbzusätzen/Pigmenten mit 2- und/oder 3-wertigen Kationen sind beispielsweise Titandioxid und Eisenoxid.

Im Rahmen der vorliegenden Erfindung bestehen die Tabletten aus einem Tablettenkern und optional hat der Tablettenkern einen Filmüberzug. Der Tablettenkern enthält den Wirkstoff (I), mindestens einen Füllstoff und mindestens ein Schmiermittel und gegebenenfalls weitere Hilfsstoffe. Bevorzugt haben die Tabletten einen Filmüberzug.

Bevorzugt haben die Tabletten einen Filmüberzug, der kein Polyethylenglykol enthält.

Die erfindungsgemäße feste, oral applizierbare pharmazeutische Darreichungsform umfasst beispielhaft und vorzugsweise Granulate, mit Granulat gefüllte Hartgelatinekapseln, Sachets oder Tabletten, bevorzugt sind Tabletten. Bevorzugt sind insbesondere den Wirkstoff (I) schnell freisetzende Tabletten.

Im Rahmen der vorliegenden Erfindung sind schnell freisetzende Tabletten insbesondere solche, die gemäß der Freisetzungsmethode des Europäischen Arzneibuchs mit Apparatur 2 (Paddle) betreffend 6 Prüfkörper nach 30 Minuten mindestens 85% Wirkstoff (I) pro Prüfkörper in das Freisetzungsmedium abgegeben haben. Die Umdrehungsgeschwindigkeit des Rührers liegt bei 50 UpM (Umdrehungen pro Minute) in dem Freisetzungsmedium bestehend aus 900 ml 0,1N Salzsäure. Diese Methode findet für schnell freisetzende Tabletten Verwendung, bei denen die Tablettendosis ≤ 100 mg (entsprechen 107 mg des Wirkstoffes (I) (Natrium-Salz)) beträgt, um sink-Bedingungen im Freisetzungsmedium zu gewährleisten. Unter Sink-Bedingungen wird das 3-fache Freisetzungsmedien-Volumen verstanden, welches benötigt würde, um mit der in der Tablette enthaltenen Wirkstoffmenge eine gesättigte Lösung herzustellen.

Besonders bevorzugt sind solche schnell freisetzenden Tabletten, die nach 1 monatiger offener Lagerung bei 40°C und 75% relativer Luftfeuchtigkeit gemäß der Freisetzungsmethode des Europäischen Arzneibuchs mit Apparatur 2 (Paddle) betreffend 6 Prüfkörper nach 30 Minuten mindestens 85% Wirkstoff (I) pro Prüfkörper in das Freisetzungsmedium abgegeben haben.

Gegenstand der vorliegenden Erfindung sind feste, oral applizierbare pharmazeutische Darreichungsformen enthaltend Natrium-1-[6-(morpholin-4-yl)pyrimidin-4-yl]-4-(1H-1,2,3-triazol-1-yl)-1H-pyrazol-5-olat (Wirkstoff (I)), dadurch gekennzeichnet, dass
(a) keine Hilfsstoffe mit 2- und/oder 3-wertigen Kationen enthalten sind,
(b) keine Laktose enthalten ist,
(c) die Konzentration des Wirkstoffes (I) ≥ 10% bezogen auf die Gesamtmasse der Formulierung beträgt,
(d) sofern die Darreichungsform einen Filmüberzug hat, dieser Filmüberzug kein Polyethylenglykol enthält, und
(e) der Wirkstoff (I) schnell freigesetzt wird.

Gegenstand der vorliegenden Erfindung sind feste, oral applizierbare pharmazeutische Darreichungsformen enthaltend Natrium-1-[6-(morpholin-4-yl)pyrimidin-4-yl]-4-(1H-1,2,3-triazol-1-yl)-1H-pyrazol-5-olat (Wirkstoff (I)), dadurch gekennzeichnet, dass
(a) keine Hilfsstoffe mit 2- und/oder 3-wertigen Kationen enthalten sind,
(b) keine Laktose enthalten ist,
(c) die Konzentration des Wirkstoffes (I) ≥ 10% bezogen auf die Gesamtmasse der Formulierung beträgt,
(d) sofern die Darreichungsform einen Filmüberzug hat, dieser Filmüberzug kein Polyethylenglykol enthält, und
(e) der Wirkstoff (I) schnell freigesetzt wird,
wobei aus der pharmazeutischen Darreichungsform betreffend 6 Prüfkörper nach 30 Minuten mindestens 85% Wirkstoff (I) pro Prüfkörper gemäß der Freisetzungsmethode des Europäischen Arzneibuchs mit Apparatur 2 (Paddle) in das Freisetzungsmedium abgegeben werden.

Der Wirkstoff (I) liegt in der Kristallmodifikation vor, in der der Wirkstoff (I) bei der Herstellung nach dem in WO 2012/065967 unter Beispiel 1 beschriebenen Weg erhalten wird und die im Rahmen der vorliegenden Erfindung als Modifikation I bezeichnet wird.

Der Wirkstoff (I) liegt in den erfindungsgemäßen pharmazeutischen Darreichungsformen in kristalliner Form vor. In einer besonders bevorzugten Ausführungsform der vorliegenden Erfindung wird der kristalline Wirkstoff (I) in mikronisierter Form der Kristallmodifikation I eingesetzt. Hierbei besitzt der Wirkstoff (I) bevorzugt eine mittlere Partikelgröße X₅₀ (50%-Anteil) kleiner 10 µm, insbesondere zwischen 1 und 8 µm, sowie einen X₉₀-Wert (90%-Anteil) kleiner 20 µm.

Der Wirkstoff (I) liegt in der erfindungsgemäßen pharmazeutischen Darreichungsform in einer Konzentration von ≥ 10% bezogen auf die Gesamtmasse der Formulierung vor, bevorzugt in einer Konzentration von 10 bis 50% bezogen auf die Gesamtmasse der Formulierung, besonders bevorzugt in einer Konzentration von 10 bis 40% bezogen auf die Gesamtmasse der Formulierung, ganz besonders bevorzugt in einer Konzentration von 15 bis 30% bezogen auf die Gesamtmasse der Formulierung.

In der erfindungsgemäßen pharmazeutischen Darreichungsform liegt keine Laktose vor.

In der erfindungsgemäßen pharmazeutischen Darreichungsform liegen keine Hilfsstoffe mit 2- und/oder 3-wertigen Kationen vor.

Gegenstand der vorliegenden Erfindung ist ein Verfahren zur Herstellung einer festen, oral applizierbaren pharmazeutischen Darreichungsform enthaltend Natrium-1-[6-(morpholin-4-yl)pyrimidin-4-yl]-4-(1H-1,2,3-triazol-1-yl)-1H-pyrazol-5-olat (Wirkstoff (I)), keine Hilfsstoffe mit 2- und/oder 3-wertigen Kationen, keine Laktose und eine Konzentration des Wirkstoffes (I) ≥ 10% bezogen auf die Gesamtmasse der Formulierung, dadurch gekennzeichnet, dass
(a) zunächst ein den Wirkstoff (I) enthaltendes Granulat hergestellt wird
(b) und das Granulat dann, gegebenenfalls unter Zusatz pharmazeutisch geeigneter Hilfsstoffe, in die pharmazeutische Darreichungsform überführt wird.

Gegenstand der vorliegenden Erfindung ist ein Verfahren zur Herstellung einer festen, oral applizierbaren pharmazeutischen Darreichungsform enthaltend Natrium-1-[6-(morpholin-4-yl)pyrimidin-4-yl]-4-(1H-1,2,3-triazol-1-yl)-1H-pyrazol-5-olat (Wirkstoff (I)), keine Hilfsstoffe mit 2- und/oder 3-wertigen Kationen, keine Laktose und eine Konzentration des Wirkstoffes (I) ≥ 10% bezogen auf die Gesamtmasse der Formulierung, dadurch gekennzeichnet, dass
(a) zunächst ein den Wirkstoff (I) enthaltendes Granulat hergestellt wird
(b) und das Granulat dann, gegebenenfalls unter Zusatz pharmazeutisch geeigneter Hilfsstoffe, in die pharmazeutische Darreichungsform überführt wird,
und wobei aus der pharmazeutischen Darreichungsform betreffend 6 Prüfkörper nach 30 Minuten mindestens 85% Wirkstoff (I) pro Prüfkörper gemäß der Freisetzungsmethode des Europäischen Arzneibuchs mit Apparatur 2 (Paddle) in das Freisetzungsmedium abgegeben werden.

Die Herstellung des Granulates im Verfahrensschritt (a) kann durch Feuchtgranulation in einem Mischer (= Mischergranulation) oder in einer Wirbelschicht (= Wirbelschichtgranulation) erfolgen oder durch Trockengranulation mittels Walzenkompaktierung, bevorzugt ist die Feuchtgranulation als Wirbelschichtgranulation.

Bei der Feuchtgranulation kann der Wirkstoff (I) entweder als Feststoff in der Vormischung (Vorlage) vorgelegt werden oder er wird in der Granulierflüssigkeit suspendiert oder er wird in einem Teil in die Vorlage und in dem anderen Teil in die Granulierflüssigkeit eingearbeitet. Bevorzugt wird der Wirkstoff (I) in der Vormischung (Vorlage) vorgelegt.

Die erfindungsgemäß verwendete Granulierflüssigkeit enthält ein Lösungsmittel und ein hydrophiles Bindemittel. Das hydrophile Bindemittel ist dabei in der Granulierflüssigkeit dispergiert oder vorzugsweise darin gelöst.

Als Lösungsmittel der Granulierflüssigkeit können organische Lösungsmittel, wie beispielsweise Ethanol oder Aceton oder Wasser oder Gemische davon verwendet werden. Bevorzugt wird Wasser als Lösungsmittel verwendet.

Als hydrophile Bindemittel werden pharmazeutisch geeignete hydrophile Zusatzstoffe eingesetzt, vorzugsweise solche, die sich im Lösungsmittel der Granulierflüssigkeit lösen. Vorzugsweise werden dabei hydrophile Polymere wie beispielsweise Hydroxypropylmethylcellulose (HPMC), Natriumcarboxymethylcellulose, Methylcellulose, Hydroxypropylcellulose (HPC), niedrig-substituierte Hydroxypropylcellulose (L-HPC), Hydroxypropylcellulose LF, Polyvinylpyrrolidon, Polyvinylalkohol, Vinylpyrrolidon-Vinylacetat-Copolymere (beispielsweise Kollidon® VA64, BASF), Gelatine, Guargummi, partiell hydrolisierte Stärke, Alginate oder Xanthan eingesetzt. Besonders bevorzugt wird Hydroxypropylmethylcellulose (HPMC) als hydrophiles Bindemittel eingesetzt.

Das hydrophile Bindemittel ist dabei in einer Konzentration von 1 bis 12% (bezogen auf die Gesamtmasse der pharmazeutischen Darreichungsform), vorzugsweise von 1 bis 6% enthalten.

In der Vormischung (Vorlage) der Feuchtgranulation sind weitere pharmazeutisch geeignete Zusatzstoffe enthalten, wie beispielsweise Füllstoffe, Trockenbindemittel und Zerfallsförderer (Sprengmittel).

Füllstoffe und Trockenbindemittel sind beispielsweise Cellulosepulver, mikrokristalline Cellulose, verkieselte mikrokristalline Cellulose, Mannitol, Maltitol, Sorbitol und Xylitol, bevorzugt mikrokristalline Cellulose oder Mannitol oder eine Mischung aus mikrokristalliner Cellulose und Mannitol.

Zerfallsförderer (Sprengmittel) sind beispielsweise Carboxymethylcellulose, Croscarmellose (quervernetzte Carboxymethylcellulose), Crospovidone (quervernetztes Polyvinylpyrrolidon), niedrig-substituierte Hydroxypropylcellulose (L-HPC), Natriumcarboxymethylstärke, Natriumglykolat der Kartoffelstärke, partiell hydrolisierte Stärke, Weizenstärke, Maisstärke, Reisstärke und Kartoffelstärke.

Das im Verfahrensschritt (a) erhaltene Granulat wird anschließend im Verfahrensschritt (b) in die erfindungsgemäße pharmazeutische Darreichungsform überführt.

Der Verfahrensschritt (b) umfasst beispielsweise Tablettieren, Abfüllen in Kapseln, vorzugsweise Hartgelatinekapseln, oder Abfüllen als Sachets, jeweils nach üblichen, dem Fachmann geläufigen Methoden, gegebenenfalls unter Zusatz weiterer pharmazeutisch geeigneter Zusatzstoffe.

Als pharmazeutisch geeignete Zusatzstoffe seien beispielsweise Schmier-, Gleit-, Fließregulierungsmittel und Zerfallsförderer (Sprengmittel) genannt.

Schmier-, Gleit-, Fließregulierungsmittel sind beispielsweise Fumarsäure, Stearinsäure, Natriumstearylfumarat, höhermolekulare Fettalkohole, Stärke (Weizen-, Reis,- Mais- oder Kartoffelstärke), Talkum, hochdisperses (kolloidales) Siliciumdioxid und Glyceroldistearat, bevorzugt Natriumstearylfumarat oder Glyceroldistearat, ganz besonders bevorzugt Natriumstearylfumarat.

Zerfallsförderer (Sprengmittel) sind beispielsweise Carboxymethylcellulose, Croscarmellose (quervernetzte Carboxymethylcellulose), Crospovidone (quervernetztes Polyvinylpyrrolidon), niedrig-substituierte Hydroxypropylcellulose (L-HPC), Natriumcarboxymethylstärke, partiell hydrolisierte Stärke, Weizenstärke, Maisstärke, Reisstärke und Kartoffelstärke.

Gegebenenfalls werden die erfindungsgemäßen Granulate oder Tabletten in einem weiteren Schritt unter üblichen, dem Fachmann geläufigen Bedingungen lackiert. Die Lackierung erfolgt unter Zusatz von üblichen, dem Fachmann geläufigen Lackier- und Filmbildemitteln wie Hydroxypropylcellulose, Hydroxypropylmethylcellulose (beispielsweise Hydroxypropylmethylcellulose 5cP oder 15 cP), Polyvinylpyrrolidon, Vinylpyrrolidon-Vinylacetat-Copolymere (beispielsweise Kollidon® VA64, BASF), Schellack, Glycerintriacetat, Triethylcitrat, Talkum und/oder Farbzusätzen/Pigmenten wie beispielsweise Titandioxid, Eisenoxide, Indigotin oder geeigneter Farblacke.

Die Feuchtgranulation wird beschrieben in:
1) W.A. Ritschel, A. Bauer-Brandl, "Die Tablette", Editio Cantor Verlag, 2. Auflage, 2002, Seiten 268-314.
2) K. H. Bauer, K.-H. Frömming, C. Führer, "Lehrbuch der pharmazeutischen Technologie", Wissenschaftliche Verlagsgesellschaft mbH Stuttgart, 6. Auflage, 1999, Seiten 305-313.

Wirkstoff (I) und Hilfsstoffe können auch gemischt und direkt tablettiert werden (Direkttablettierung).

Bevorzugt ist die Tablettierung über das zunächst hergestellte Granulat.

Weiterhin wurde im Rahmen der Tablettenentwicklung überraschenderweise festgestellt, dass sich bei Lichteinwirkung auf den Wirkstoff (I) oder die Tablettenkerne diese ins bräunliche verfärben. Damit ist eine Lackierung der Arzneiform für ausreichenden Lichtschutz vorteilhaft.

Weiterer Gegenstand der vorliegenden Erfindung sind Arzneimittel, enthaltend eine erfindungsgemäße feste, oral applizierbare pharmazeutische Darreichungsform enthaltenden den Wirkstoff (I).

Weiterer Gegenstand der vorliegenden Erfindung ist die Verwendung der erfindungsgemäßen festen, oral applizierbaren pharmazeutischen Darreichungsform enthaltenden den Wirkstoff (I), zur Herstellung eines Arzneimittels zur Prophylaxe, Sekundärprophylaxe und/oder Behandlung von Erkrankungen, insbesondere von Herz-Kreislauf-Erkrankungen, Herzinsuffizienz, Anämie, chronischen Nierenerkrankungen und Niereninsuffizienz.

Weiterer Gegenstand der vorliegenden Erfindung ist die Verwendung der erfindungsgemäßen festen, oral applizierbaren pharmazeutischen Darreichungsform enthaltend den Wirkstoff (I) zur Prophylaxe, Sekundärprophylaxe und/oder Behandlung von Erkrankungen, insbesondere von Herz-Kreislauf-Erkrankungen, Herzinsuffizienz, Anämie, chronischen Nierenerkrankungen und Niereninsuffizienz.

Weiterer Gegenstand der vorliegenden Erfindung ist die Verwendung von Natrium-1-[6-(morpholin-4-yl)pyrimidin-4-yl]-4-(1H-1,2,3-triazol-1-yl)-1H-pyrazol-5-olat (I) zur Herstellung einer erfindungsgemäßen festen, oral applizierbaren pharmazeutischen Darreichungsform.

Weiterer Gegenstand der vorliegenden Erfindung ist ein Verfahren zur Prophylaxe, Sekundärprophylaxe und/oder Behandlung von Herz-Kreislauf-Erkrankungen, Herzinsuffizienz, Anämie, chronischen Nierenerkrankungen und Niereninsuffizienz durch Verabreichung einer erfindungsgemäßen festen, oral applizierbaren, den Wirkstoff (I) enthaltende pharmazeutischen Darreichungsform.

Die Erfindung wird nachstehend durch bevorzugte Ausführungsbeispiele näher erläutert, auf welche sie jedoch nicht eingeschränkt ist. Soweit nicht anders angegeben, beziehen sich nachstehend alle Mengenangaben auf Gewichtsprozente.

### Experimenteller Teil:

### 1. Freisetzungsmethode

Gemäß Europäischem Arzneibuch, 6. Ausgabe, Grundwerk 2008 wird die Arzneiform mit Apparatur 2 (Paddle) geprüft. Die Umdrehungsgeschwindigkeit des Rührers liegt bei 50 UpM (Umdrehungen pro Minute) in 900 ml 0,1N Salzsäure. Das Freisetzungskriterium ist dann erfüllt, wenn alle 6 Prüfkörper nach einem Untersuchungszeitraum von 30 Minuten mindestens 85% Wirkstoff (I) in das Freisetzungsmedium abgegeben haben. Diese Methode findet für schnell freisetzende Tabletten Verwendung, bei denen die Tablettendosis ≤ 100 mg (entsprechen 107 mg des Wirkstoffes (I) (Natrium-Salz)) beträgt, um sink-Bedingungen im Freisetzungsmedium zu gewährleisten. Unter Sink-Bedingungen wird das 3-fache Freisetzungsmedien-Volumen verstanden, welches benötigt würde, um mit der in der Tablette enthaltenen Wirkstoffmenge eine gesättigte Lösung herzustellen.

### 2. Bestimmung der Bruchfestigkeit

Gemäß Europäischem Arzneibuch, 6. Ausgabe, Grundwerk 2008 wird die Kraft gemessen, die notwendig ist, um Tabletten durch Druck zu zerbrechen. Das Messgerät besteht aus zwei Backen, die sich gegenüber stehen. Einer der Backen bewegt sich auf den anderen zu. Die Backenflächen sind flach und größer als die Kontaktzone zur Tablette sowie senkrecht zur Bewegungsrichtung angeordnet. Das Gerät wird mit einem System, das eine Genauigkeit von etwa 1 Newton aufweist, kalibriert. Die Tablette wird zwischen die Backen gelegt, wobei ggf. die Form, die Bruchrille und die Prägung berücksichtigt werden. Bei jeder Messung wird die Tablette in gleicher Weise in Bezug auf die Richtung der Kraft orientiert. Die Prüfung wird an 10 Tabletten durchgeführt. Tablettenfragmente müssen vor jeder Prüfung entfernt werden.

### 3. Herstellverfahren Wirbelschichtgranulation

### Beispiele 6-1 (Vergleichsbeispiel), 6-2 (Vergleichsbeispiel), 6-8 (Vergleichsbeispiel), 6-9 und 6-10

Das Bindemittel wird in Wasser gelöst und der Wirkstoff (I) in dieser Lösung suspendiert. Diese Suspension wird als Granulierflüssigkeit im Rahmen einer Wirbelschichtgranulation auf die Vorlage bestehend aus Füllstoffen, gegebenenfalls Laktose und 50% Zerfallsförderer aufgesprüht. Nach Trocknung und Siebung (0,8 mm Maschenweite) des entstandenen Granulates werden die übrigen 50% Zerfallsförderer und ein Schmiermittel, welches gegebenenfalls auch Magnesiumstearat ist, zugegeben und gemischt. Das so erhaltene pressfertige Granulat wird zu Tabletten verpresst. Die anschließende Lackierung der Tabletten erfolgt mit Pigmenten, die in einer wässrigen Lösung bestehend aus Lackier- und Filmbildemitteln und gegebenenfalls Polyethylenglykol suspendiert sind.

### Beispiel 6-3 (Vergleichsbeispiel)

Das Bindemittel wird in Wasser gelöst und der Wirkstoff (I) in dieser Lösung suspendiert. Diese Suspension wird als Granulierflüssigkeit im Rahmen einer Wirbelschichtgranulation auf die Vorlage bestehend aus Füllstoffen, Laktose und Zerfallsförderer aufgesprüht. Nach Trocknung und Siebung (0,8 mm Maschenweite) des entstandenen Granulates wird ein Schmiermittel zugegeben und gemischt. Das so erhaltene pressfertige Granulat wird zu Tabletten verpresst.

### Beispiele 6-4 (Vergleichsbeispiel) und 6-5 (Vergleichsbeispiel)

Das Bindemittel wird in Wasser gelöst. Diese Bindemittellösung wird als Granulierflüssigkeit im Rahmen einer Wirbelschichtgranulation auf die Vorlage bestehend aus Wirkstoff (I) und Füllstoffen und gegebenenfalls Laktose aufgesprüht. Nach Trocknung und Siebung (0,8 mm Maschenweite) des entstandenen Granulates werden Zerfallsförderer und Magnesiumstearat zugegeben und gemischt. Das so erhaltene pressfertige Granulat wird zu Tabletten verpresst.

### Beispiele 6-6 (Vergleichsbeispiel) und 6-7 (Vergleichsbeispiel)

Das Bindemittel wird in Wasser gelöst und 50% des Wirkstoffes (I) in dieser Lösung suspendiert. Diese Suspension wird als Granulierflüssigkeit im Rahmen einer Wirbelschichtgranulation auf die Vorlage bestehend aus 50% Wirkstoff (I) und Füllstoffen und 50% Zerfallsförderer aufgesprüht. Nach Trocknung und Siebung (0,8 mm Maschenweite) des entstandenen Granulates werden die übrigen 50% Zerfallsförderer und Magnesiumstearat zugegeben und gemischt. Das so erhaltene pressfertige Granulat wird zu Tabletten verpresst. Die anschließende Lackierung der Tabletten erfolgt mit Pigmenten, die in einer wässrigen Lösung bestehend aus Lackier- und Filmbildemitteln und Polyethylenglykol suspendiert sind.

### Beispiele 6-11, 6-12, 6-15, 6-16 (Vergleichsbeispiel), 6-17 (Vergleichsbeispiel), 6-18 und 6-19 (Vergleichsbeispiel)

Das Bindemittel wird in Wasser gelöst. Diese Bindemittellösung wird als Granulierflüssigkeit im Rahmen einer Wirbelschichtgranulation auf die Vorlage bestehend aus Wirkstoff (I) und Füllstoffen, gegebenenfalls Laktose und Zerfallsförderer aufgesprüht. Nach Trocknung und Siebung (0,8 mm Maschenweite) des entstandenen Granulates wird ein Schmiermittel zugegeben und gemischt. Das so erhaltene pressfertige Granulat wird zu Tabletten verpresst. Die gegebenenfalls anschließende Lackierung der Tabletten erfolgt mit Pigmenten, die in einer wässrigen Lösung bestehend aus Lackier- und Filmbildemitteln suspendiert sind.

### Beispiel 6-21

Das Bindemittel wird in Wasser gelöst. Diese Bindemittellösung wird als Granulierflüssigkeit im Rahmen einer Wirbelschichtgranulation auf die Vorlage bestehend aus Wirkstoff (I), den Füllstoffen und dem Zerfallsförderer aufgesprüht. Nach Trocknung und Siebung (0,8 mm Maschenweite) des entstandenen Granulates wird in einem zweistufigen Prozess zunächst das Gleitmittel und dann das Schmiermittel zugegeben und gemischt. Das so erhaltene pressfertige Granulat wird zu Tabletten verpresst. Die anschließende Lackierung der Tabletten erfolgt mit Pigmenten, die in einer wässrigen Lösung bestehend aus Lackier- und Filmbildemitteln suspendiert sind.

### 4. Herstellverfahren Mischergranulation

### Beispiele 6-13 (Vergleichsbeispiel) und 6-14 (Vergleichsbeispiel)

In einem Schnellmischer werden Wirkstoff (I), Füllstoffe und ca. 40% des Zerfallsförderers gemischt (Granulatvorlage). Eine 3%ige Bindemittellösung wird hergestellt und als Granulierflüssigkeit der Granulatvorlage zugegeben. Das Ganze wird mit Hilfe des schnell rotierenden Rührwerkes gleichmäßig gemischt. Nach erfolgter Durchmischung wird das feuchte Granulat gesiebt (2 mm Maschenweite) und getrocknet. Nach Siebung des getrockneten Granulates (0,8 mm Maschenweite) wird mit ca. 60% Zerfallsförderer und Magnesiumstearat nachgemischt, was in zwei separaten Mischungsschritten erfolgt. Das so erhaltene pressfertige Granulat wird zu Tabletten verpresst.

### 5. Herstellverfahren Walzenkompaktierung

### Beispiel 6-20 (Vergleichsbeispiel)

Wirkstoff (I), Füllstoff, Zerfallsförderer und Trockenbindemittel werden in einem Freifallmischer gemischt. Die Pulvermischung wird gesiebt (1,0 mm Maschenweite) und anschließend erneut in einem Freifallmischer gemischt. Gesiebtes hochdisperses Siliciumdioxid wird zugegeben und durch Mischen homogen verteilt. Vor dem letzten Mischungsschritt wird Magnesiumstearat zugegeben. Die so erhaltene finale Mischung wird mittels Walzenkompaktierung trocken granuliert und das Granulat im Anschluß zu Tabletten verpresst.

**Abkürzungen:**

| | |
|---|---|
| Hydroxypropylmethylcellulose 5 cP (HPMC 5cP) | eine 2%ige wäßrige Lösung von HPMC 5cP weist bei 20°C eine Viskosität von 5 mPas auf |
| Hydroxypropylcellulose LF (HPC LF) | eine 5%ige wäßrige Lösung von HPC LF weist bei 20°C eine Viscosität von 75-150 mPas auf |
| Hydroxypropylmethylcellulose 3 cP (HPMC 3cP) | eine 2%ige wäßrige Lösung von HPMC 3cP weist bei 20°C eine Viskosität von 2,4-3,6 mPas auf |
| WR | Wölbungsradius |
| MW | Mittelwert |
| r.F. | relative Luftfeuchtigkeit |
| N | Newton |

### 6. Zusammensetzungen der Darreichungsform in mg/Tablette

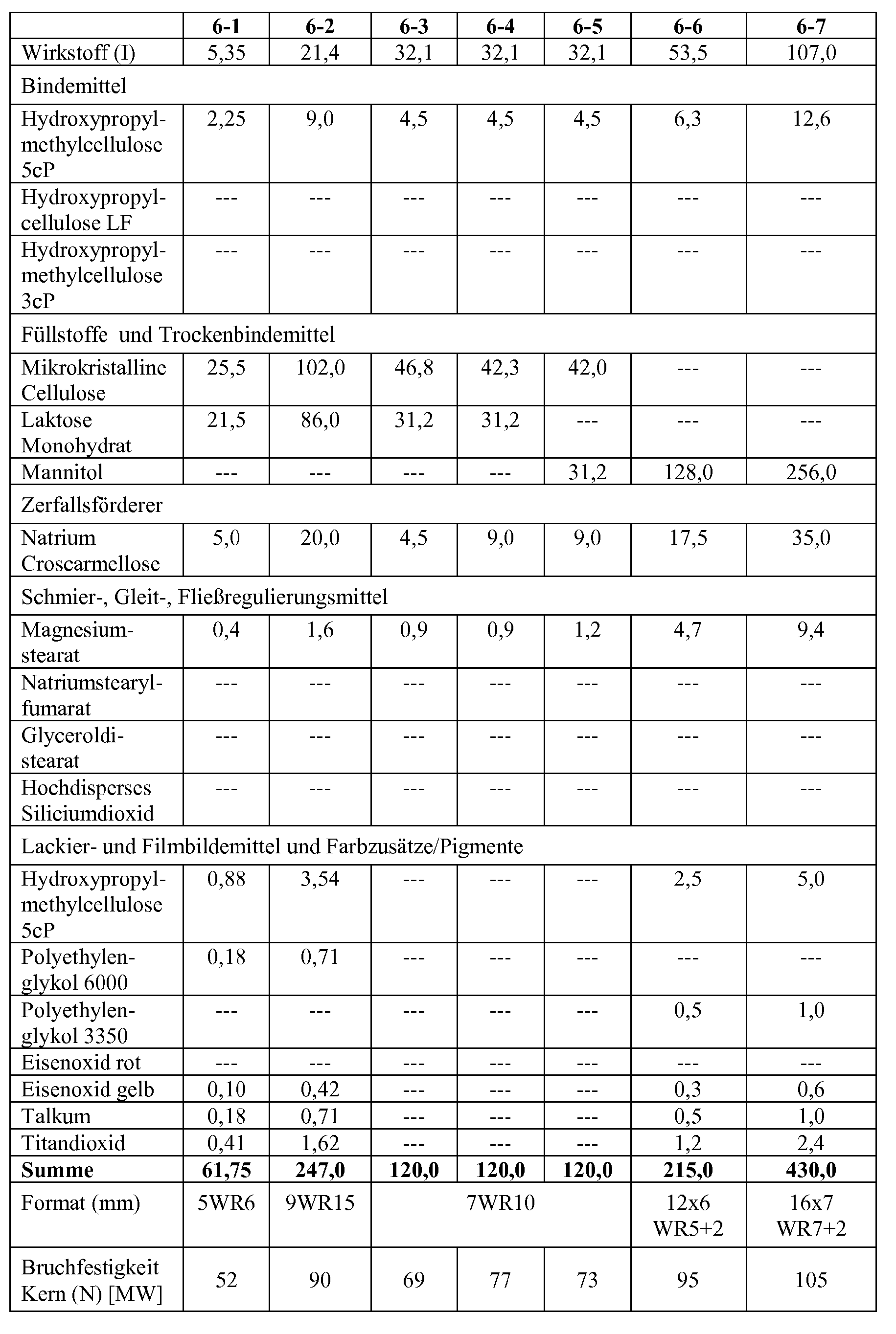

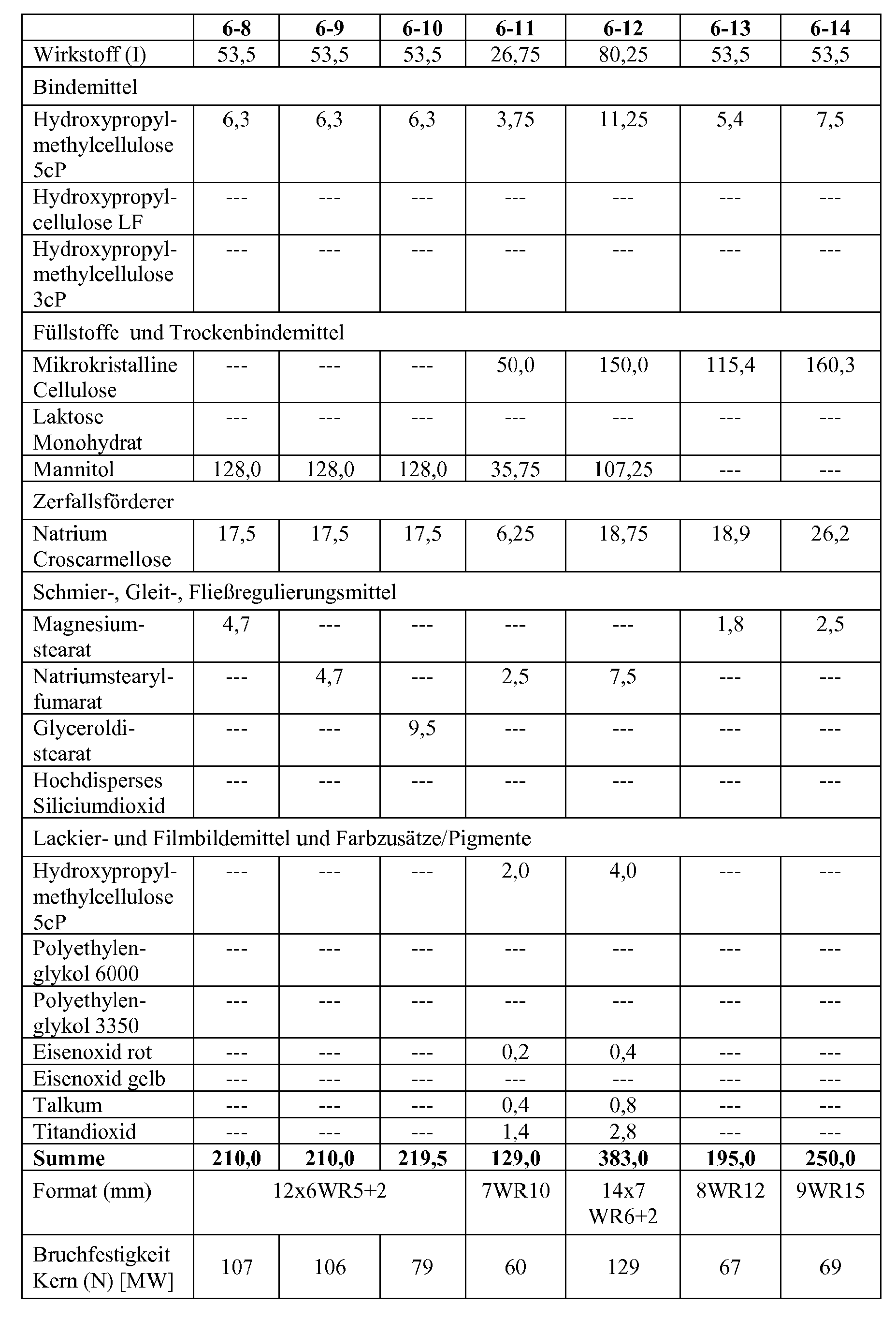

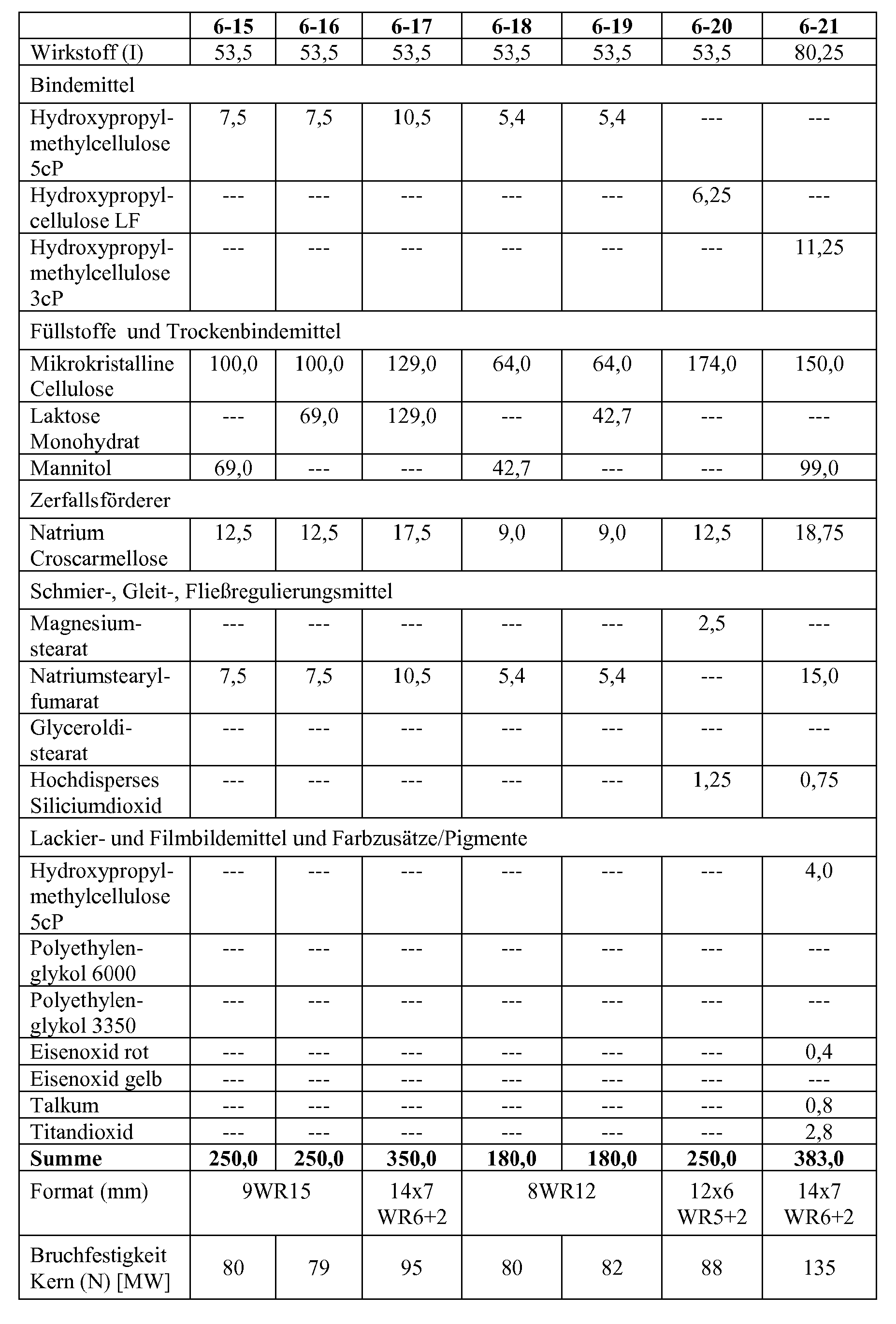

### 7. Freisetzungsergebnisse nach Herstellung der Tablette

| **Tablette gemäß Beispiel** | **Freisetzung nach 30 Min Min/Max / Mittelwert (n=6)** | **Freisetzungskriterium erfüllt** |
|---|---|---|
| **6-1** | 100/104/101 | ja |
| **6-2** | 94/99/98 | ja |
| **6-3** | 38/69/59 | nein |
| **6-4** | 64/74/71 | nein |
| **6-5** | 87/91/90 | ja |
| **6-6** | 91/96/94 | ja |
| **6-7** | 91/95/94 | ja |
| **6-8** | 98/100/98 | ja |
| **6-9** | 99/100/99 | ja |
| **6-10** | 93/96/95 | ja |
| **6-11** | 99/101/99 | ja |
| **6-12** | 88/97/94 | ja |
| **6-13** | 93/95/94 | ja |
| **6-14** | 94/97/96 | ja |
| **6-15** | 94/95/94 | ja |
| **6-16** | 84/95/91 | nein |
| **6-17** | 76/92/87 | nein |
| **6-18** | 93/95/95 | ja |
| **6-19** | 81/92/86 | nein |
| **6-20** | 93/97/95 | ja |
| **6-21** | 85/96/91 | ja |

### 8. Freisetzungsergebnisse nach Stresstest der Tablette

| **Tablette gemäß Beispiel** | **Freisetzung nach 30 Min Min/Max / Mittelwert (n=6)** | **Freisetzungskriterium erfüllt** | **Bedingungen** |
|---|---|---|---|
| **6-1** | 98/103/101 | ja | 1 Monat 40°C/75% r.F., in Flasche ohne Deckel (offene Lagerung) |
| **6-6** | 4/20/12 | nein | 1 Monat 40°C/75% r.F., in Flasche ohne Deckel (offene Lagerung) |
| **6-8** | 80/91/86 | nein | 1 Monat 40°C/75% r.F., in Flasche ohne Deckel (offene Lagerung) |
| **6-9** | 97/99/99 | ja | 1 Monat 40°C/75% r.F., in Flasche ohne Deckel (offene Lagerung) |
| **6-10** | 91/97/93 | ja | 1 Monat 40°C/75% r.F., in Flasche ohne Deckel (offene Lagerung) |
| **6-11** | 90/97/94 | ja | 1 Monat 40°C/75% r.F., in Flasche ohne Deckel (offene Lagerung) |
| **6-12** | 89/95/93 | ja | 1 Monat 40°C/75% r.F., in Flasche ohne Deckel (offene Lagerung) |

Die Beispiele 6-1 und 6-2 sind Tabletten mit niedrigen Konzentrationen an Wirkstoff (I) und dienen als Vergleichsbeispiele. In diesen Beispielen liegt die Konzentration an Wirkstoff (I) unter 10% bezogen auf die Gesamtmasse der Formulierung. Diese Tabletten zeigen die gewünschten Freisetzungseigenschaften.

Erhöht man die Konzentration an Wirkstoff (I) in der Tablette aus Beispiel 6-1 um das Dreifache, wonach sich die Tablette aus Beispiel 6-3 ergibt, so erfüllt diese Tablette aus Beispiel 6-3 nicht die Freisetzungseigenschaften nach der Herstellung der Tablette.

Leichte Variationen des Anteils an Zerfallsförderer wie in Beispiel 6-4 gezeigt, ändern die Freisetzungscharakteristika der Tablette nicht. Einen deutlichen Einfluss auf die Freisetzungseigenschaften sieht man nach Austausch des Füllstoffs Laktose Monohydrat durch Mannitol (Beispiel 6-5) bzw. durch alleinigen Einsatz von Mannitol als Füllstoff (Beispiele 6-6 und 6-7). Obwohl diese drei Formulierungen weiterhin Magnesiumstearat als Schmiermittel und Beispiele 6-6 und 6-7 auch Polyethylenglykol im Lack enthalten, ist die Freisetzung der Tabletten nach Herstellung gut. Hier macht sich der negative Einfluss des Magnesiumstearates und des Polyethylenglykols erst im Laufe des Stresstests bemerkbar (Beispiel 6-6).

Anhand der Beispiele 6-8, 6-9 und 6-10 wird der Einfluss des Magnesiumstearats innerhalb einer Lagerung unter Feuchtestress deutlich. Alle drei Tablettenbeispiele sind Laktose- und Polyethylenglykol-frei; sie variieren ausschließlich in der Art des Schmiermittels. Nur die Magnesiumstearat-haltige Formulierung erfüllt die Freisetzungsanforderungen nach einem Monat Lagerung nicht (Beispiel 6-8).

Die Beispiele 6-11 und 6-12 enthalten ca. 20% Wirkstoff (I) im Granulat, keine Laktose und kein Magnesiumstearat im Tablettenkern und kein Polyethylenglykol im Filmüberzug. Sie erfüllen die Freisetzungsanforderungen sowohl nach Herstellung als auch nach 1 monatiger offener Lagerung unter Feuchtestress.

Die Beispiele 6-13 und 6-14 belegen die Eignung der Mischergranulation als zweites Feuchtgranulationsverfahren zur Herstellung von Granulaten/Tabletten mit Wirkstoff (I). Direkt nach Herstellung zeigen die Tabletten das gewünschte Freisetzungsprofil.

Die Beispiele 6-16, 6-17 und 6-19 untersuchen systematisch den Einfluss der Wirkstoffkonzentration im Granulat (zwischen 15% und 30%) unter Einsatz von Laktose Monohydrat als Hilfsstoff. Selbst die verdünnteste Formulierung (Beispiel 6-17) zeigt nach Herstellung der Tabletten nicht die gewünschten Freisetzungscharakteristika.

Im Gegensatz dazu zeigen die Formulierungen 6-15, 6-18 und 6-21, in denen Laktose Monohydrat gegen Mannitol ausgetauscht wurde, die angestrebten Freisetzungsprofile.

Beispiel 6-20 belegt die Eignung der Trockengranulation als weiteres Granulationsverfahren zur Herstellung von Granulaten/Tabletten mit Wirkstoff (I). Nach Herstellung zeigen die Tabletten das gewünschte Freisetzungsprofil.

Wie die Daten in den Tabellen zur Freisetzung nach der Herstellung der Tabletten (unter 7.) und zur Freisetzung nach dem Stresstest der Tabletten (unter 8.) zeigen, weisen überraschenderweise nur die Tabletten mit hohen Konzentrationen an Wirkstoff (I) das gewünschte Freisetzungsprofil auf, die keine Laktose, kein Magnesiumstearat und kein Polyethylenglykol enthalten. Dieses sind die Tabletten aus den Beispielen 6-9, 6-10, 6-11, 6-12, 6-15, 6-18 und 6-21.

Diese überraschenden Eigenschaften sind am besten beim Vergleich der Tabletten aus den Beispielen 6-6, 6-8 und 6-16, die nicht das gewünschte Freisetzungsprofil zeigen, mit den Tabletten aus den Beispielen 6-9 und 6-10 zu erkennen.

## Patentansprüche

1. Feste, oral applizierbare pharmazeutische Darreichungsform enthaltend Natrium-1-[6-(morpholin-4-yl)pyrimidin-4-yl]-4-(1H-1,2,3-triazol-1-yl)-1H-pyrazol-5-olat (Wirkstoff (I)), **dadurch gekennzeichnet, dass**
(a) keine Hilfsstoffe mit 2- und/oder 3-wertigen Kationen enthalten sind,
(b) keine Laktose enthalten ist,
(c) die Konzentration des Wirkstoffes (I) ≥ 10% bezogen auf die Gesamtmasse der Formulierung beträgt, und
(d) sofern die Darreichungsform einen Filmüberzug hat, dieser Filmüberzug kein Polyethylenglykol enthält.

2. Feste, oral applizierbare pharmazeutische Darreichungsform gemäß Anspruch 1, **dadurch gekennzeichnet, dass**
(e) der Wirkstoff (I) schnell freigesetzt wird.

3. Feste, oral applizierbare pharmazeutische Darreichungsform gemäß Anspruch 2, **dadurch gekennzeichnet, dass** aus der pharmazeutischen Darreichungsform betreffend 6 Prüfkörper nach 30 Minuten mindestens 85% Wirkstoff (I) pro Prüfkörper gemäß der Freisetzungsmethode des Europäischen Arzneibuchs mit Apparatur 2 (Paddle) in das Freisetzungsmedium abgegeben werden.

4. Feste, oral applizierbare pharmazeutische Darreichungsform gemäß Anspruch 3, **dadurch gekennzeichnet, dass** das Freisetzungsmedium aus 900 ml 0,1N Salzsäure besteht.

5. Feste, oral applizierbare pharmazeutische Darreichungsform gemäß Anspruch 1 bis 4, **dadurch gekennzeichnet, dass** die Darreichungsform eine Tablette ist.

6. Feste, oral applizierbare pharmazeutische Darreichungsform gemäß Anspruch 1 bis 5, **dadurch gekennzeichnet, dass** die Konzentration des Wirkstoffes (I) 10 bis 40% bezogen auf die Gesamtmasse der Formulierung beträgt.

7. Feste, oral applizierbare pharmazeutische Darreichungsform gemäß Anspruch 1 bis 6, **dadurch gekennzeichnet, dass** als Hilfsstoffe mindestens ein Füllstoff und mindestens ein Schmiermittel enthalten sind.

8. Feste, oral applizierbare pharmazeutische Darreichungsform gemäß Anspruch 7, **dadurch gekennzeichnet, dass** der Füllstoff mikrokristalline Cellulose oder Mannitol oder eine Mischung aus mikrokristalliner Cellulose und Mannitol ist.

9. Feste, oral applizierbare pharmazeutische Darreichungsform gemäß Anspruch 7, **dadurch gekennzeichnet, dass** das Schmiermittel Natriumstearylfumarat oder Glyceroldistearat ist.

10. Feste, oral applizierbare pharmazeutische Darreichungsform gemäß Anspruch 5 bis 9, **dadurch gekennzeichnet, dass** die Tablette einen Filmüberzug hat.

11. Verfahren zur Herstellung einer festen, oral applizierbaren pharmazeutischen Darreichungsform enthaltend Natrium-1-[6-(morpholin-4-yl)pyrimidin-4-yl]-4-(1H-1,2,3-triazol-1-yl)-1H-pyrazol-5-olat (Wirkstoff (I)), keine Hilfsstoffe mit 2- und/oder 3-wertigen Kationen, kein Laktose und eine Konzentration des Wirkstoffes (I) ≥ 10% bezogen auf die Gesamtmasse der Formulierung, **dadurch gekennzeichnet, dass**
(a) zunächst ein den Wirkstoff (I) enthaltendes Granulat hergestellt wird
(b) und das Granulat dann, gegebenenfalls unter Zusatz pharmazeutisch geeigneter Hilfsstoffe, in die pharmazeutische Darreichungsform überführt wird.

12. Verfahren gemäß Anspruch 11, **dadurch gekennzeichnet, dass** das Granulat durch Feuchtgranulation hergestellt wird.

13. Verfahren gemäß Anspruch 12, **dadurch gekennzeichnet, dass** als Feuchtgranulationsmethode die Wirbelschichtgranulation verwendet wird.

## Claims

1. Solid pharmaceutical dosage form for oral administration comprising sodium 1-[6-(morpholin-4-yl)pyrimidin-4-yl]-4-(1H-1,2,3-triazol-1-yl)-1H-pyrazol-5-olate (active ingredient (I)), **characterized in that**
(a) no auxiliaries having divalent and/or trivalent cations are present,
(b) no lactose is present,
(c) the concentration of active ingredient (I) is ≥ 10% based on the total mass of the formulation, and
(d) if the dosage form has a film coating, said film coating does not comprise polyethylene glycol.

2. Solid pharmaceutical dosage form for oral administration according to Claim 1, **characterized in that**
(e) the active ingredient (I) is released rapidly.

3. Solid pharmaceutical dosage form for oral administration according to Claim 2, **characterized in that** at least 85% of active ingredient (I) are released per test specimen into the release medium after 30 minutes, from 6 test specimens relating to the pharmaceutical dosage form, according to the release method of the European Pharmacopoeia using apparatus 2 (paddle).

4. Solid pharmaceutical dosage form for oral administration according to Claim 3, **characterized in that** the release medium consists of 900 ml of 0.1N hydrochloric acid.

5. Solid pharmaceutical dosage form for oral administration according to Claims 1 to 4, **characterized in that** the dosage form is a tablet.

6. Solid pharmaceutical dosage form for oral administration according to Claims 1 to 5, **characterized in that** the concentration of the active ingredient (I) is 10 to 40% based on the total mass of the formulation.

7. Solid pharmaceutical dosage form for oral administration according to Claims 1 to 6, **characterized in that** at least one filler and at least one lubricant are present as auxiliaries.

8. Solid pharmaceutical dosage form for oral administration according to Claim 7, **characterized in that** the filler is microcrystalline cellulose or mannitol or a mixture of microcrystalline cellulose and mannitol.

9. Solid pharmaceutical dosage form for oral administration according to Claim 7, **characterized in that** the lubricant is sodium stearyl fumarate or glyceryl distearate.

10. Solid pharmaceutical dosage form for oral administration according to Claims 5 to 9, **characterized in that** the tablet has a film coating.

11. Method for preparing a solid pharmaceutical dosage form for oral administration comprising sodium 1-[6-(morpholin-4-yl)pyrimidin-4-yl]-4-(1H-1,2,3-triazol-1-yl)-1H-pyrazol-5-olate (active ingredient (I)), no auxiliaries having divalent and/or trivalent cations, no lactose and a concentration of active ingredient (I) ≥ 10% based on the total mass of the formulation, **characterized in that**
(a) a granulate comprising the active ingredient (I) is initially prepared
(b) and the granulate, optionally with addition of pharmaceutically acceptable auxiliaries, is then converted into the pharmaceutical dosage form.

12. Method according to Claim 11, **characterized in that** the granulate is prepared by wet granulation.

13. Method according to Claim 12, **characterized in that** fluidized bed granulation is used as the wet granulation method.

## Revendications

1. Forme galénique pharmaceutique solide applicable par voie orale contenant du 1-[6-(morpholin-4-yl)pyrimidin-4-yl]-4-(1H-1,2,3-triazol-1-yl)-1H-pyrazol-5-olate de sodium (principe actif (I)), **caractérisée en ce que**
a) aucune substance auxiliaire comportant des cations divalents et/ou trivalents n'est contenue,
b) aucun lactose n'est contenu,
c) la concentration du principe actif (I) est ≥ 10 %, par rapport à la masse totale de la formulation, et
d) si la forme galénique possède un pelliculage, ce pelliculage ne contient pas de polyéthylèneglycol.

2. Forme galénique pharmaceutique solide applicable par voie orale selon la revendication 1, **caractérisé en ce que**
(e) le principe actif (I) est rapidement libéré.

3. Forme galénique pharmaceutique solide applicable par voie orale selon la revendication 2, **caractérisée en ce que**, de 6 éprouvettes relatives à la forme galénique pharmaceutique, au moins 85 % de principe actif (I) sont libérés dans le milieu de libération par éprouvette après 30 minutes, selon le procédé de libération de la Pharmacopée Européenne avec l'appareillage 2 (spatule).

4. Forme galénique pharmaceutique solide applicable par voie orale selon la revendication 3, **caractérisée en ce que** le milieu de libération est constitué de 900 ml d'acide chlorhydrique 0,1 N.

5. Forme galénique pharmaceutique solide applicable par voie orale selon les revendications 1 à 4, **caractérisée en ce que** la forme galénique est un comprimé.

6. Forme galénique pharmaceutique solide applicable par voie orale selon les revendications 1 à 5, **caractérisée en ce que** la concentration du principe actif (I) est de 10 à 40 % par rapport à la masse totale de la formulation.

7. Forme galénique pharmaceutique solide applicable par voie orale selon les revendications 1 à 6, **caractérisée en ce qu'**en tant que substances auxiliaires, au moins une charge et au moins un agent glissant sont contenus.

8. Forme galénique pharmaceutique solide applicable par voie orale selon la revendication 7, **caractérisée en ce que** la charge est de la cellulose microcristalline ou du mannitol ou un mélange de cellulose microcristalline et de mannitol.

9. Forme galénique pharmaceutique solide applicable par voie orale selon la revendication 7, **caractérisée en ce que** l'agent glissant est du fumarate de stéaryle et de sodium ou du distéarate de glycérol.

10. Forme galénique pharmaceutique solide applicable par voie orale selon les revendications 5 à 9, **caractérisée en ce que** le comprimé possède un pelliculage.

11. Procédé pour la préparation d'une forme galénique pharmaceutique solide applicable par voie orale contenant du 1-[6-(morpholin-4-yl)pyrimidin-4-yl]-4-(1H-1,2,3-triazol-1-yl)-1H-pyrazol-5-olate de sodium (principe actif (I)), aucune substance auxiliaire comportant des cations divalents et/ou trivalents, pas de lactose et une concentration du principe actif (I) ≥ 10 %, par rapport à la masse totale de la formulation, **caractérisé en ce que**
(a) un granulé contenant le principe actif (I) est d'abord préparé
(b) et le granulé est ensuite converti en sa forme galénique pharmaceutique, éventuellement avec ajout de substances auxiliaires pharmaceutiquement acceptables.

12. Procédé selon la revendication 11, **caractérisé en ce que** le granulé est préparé par granulation humide.

13. Procédé selon la revendication 12, **caractérisé en ce que** la granulation en couche tourbillonnaire est utilisée en tant que procédé de granulation humide.
